## Europäisches Patentamt

## European Patent Office

## Office européen des brevets

(11) Veröffentlichungsnummer: **0 316 260**
**A2**

## (12) EUROPÄISCHE PATENTANMELDUNG

(21) Anmeldenummer: **88730229.7**

(22) Anmeldetag: **08.10.88**

(51) Int. Cl.4: **G 01 N 31/00**

(30) Priorität: **09.11.87 DE 3738353**

(43) Veröffentlichungstag der Anmeldung:
**17.05.89 Patentblatt 89/20**

(84) Benannte Vertragsstaaten:
**AT BE CH DE FR GB IT LI NL SE**

(71) Anmelder: **HARBAUER GmbH & CO KG Ingenieursbüro für Umwelttechnik**
**Bismarckstrasse 10-12**
**D-1000 Berlin 12 (DE)**

(72) Erfinder: **Kropp, Bernhard, Dr.**
**Palmzeile 7**
**D-1000 Berlin 38 (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) Verfahren zur analytischen Bestimmung des Gehaltes an chlorierten Kohlenwasserstoffen (CKW).

(57) Verfahren zur analytischen Bestimmung des Gehalts an chlorierten, gegebenenfalls entsprechend auch bromierten oder jodierten, Kohlenwasserstoffen (CKW), in einer zu analysierenden Flüssigkeit, mit folgenden Schritten: Zuführung der Flüssigkeit in ununterbrochenem Fluß unter Vermischung mit einem, insbesondere organischen, Extraktionsmittel unter dosierender, kontinuierlicher und stoßfreier Pumpwirkung; nachfolgende Phasentrennung, Vergasung, Verbrennung sowie Kondensation des organischen Anteils; quantitative kontinuierliche Bestimmung des mit den nachzuweisenden CKWs in stöchiometrischem Verhältnis stehenden Chlors durch Einleitung in eine coulombmetrische Meßzelle, sowie die Rückrechnung vom jeweils für einen Bezugszeitraum ermittelten Chlor auf die in dem entsprechenden Bezugszeitraum angefallenen chlorierten Kohlenwasserstoffe.

Fig. 1

EP 0 316 260 A2

**Beschreibung**

## Verfahren zur analytischen Bestimmung des Gehalts an chlorierten Kohlenwasserstoffen (CKW)

Die Erfindung betrifft ein Verfahren der im Oberbegriff des Anspruchs 1 angegebenen Art sowie eine Vorrichtung zur Durchführung des Verfahrens.

Zum Nachweis von chlorierten Kohlenwasserstoffen (CKW) sind die sogenannten AOX-, EOX- und POX-Verfahren bekannt. Diese Verfahren bilden diskontinuierliche Einzelnachweise, welche nur zur Verarbeitung von manuell in diskreten Mengen entnommenen Einzelproben geeignet sind. Dabei hat das AOX-Verfahren in der Normung Niederschlag gefunden, während das EOX-Verfahren in eine allgemeine Laborvorschrift eingegangen ist.

Das Erfordernis der Entnahme einer jeweils begrenzten Probenmenge in mehr oder weniger bestimmten Zeitabständen ist sehr arbeitsintensiv und erfordert eine große Zahl von gleichbleibenden sich immer wiederholenden Arbeitsvorgängen. Die diskreten Volumina von Meßlösungen und Reagenzien müssen darüberhinaus jeweils genau abgemessen und kontrolliert zusammengeführt werden. Das Laborpersonal ist hierbei den zu bearbeitenden Chemikalien ausgesetzt.

Beim bekannten EOX-Verfahren wird in einer ersten Stufe die zu analysierende Probe in ein Extraktionsmittel wie Benzin, Hexan oder ein anderes Lösungsmittel eingeleitet, welches bevorzugt chlorierte Kohlenwasserstoffe aufnimmt. Hierdurch werden bereits eine große Anzahl störender Anteile in der Probe zurückgehalten.

Anschließend wird eine Phasentrennung vorgenommen, wobei das die CKW enthaltende Extraktionsmittel von der wäßrigen Phase getrennt wird.

Bei der nachfolgenden Verbrennung des die gelösten Stoffe enthaltenden Extraktionsmittels werden die hier in Hexan gelösten Stoffe in anorganische Chloride überführt. Man spricht hier auch von einer "Mineralisierung".

Nach der Mineralisierung wird über die entstandene Salzsäure der Cl-Anteil detektiert. Dieser Nachweis kann elektrisch oder photometrisch erfolgen.

Bei dem bisher manuellen Nachweis im Reagenzglas mit diskontinuierlicher Arbeitsweise war die Phasentrennung manuell in einem Scheidetrichter durchzuführen, während die Verbrennung des die CKW enthaltenen Extraktionsmittels unter Sauerstoffatmosphäre in einem Schiffchen innerhalb eines Röhrenofens vorgenommen werden mußte. Als Alternative steht nur noch die Verbrennung in einer ständig brennenden Knallgasflamme zur Verfügung.

Nach dem sich daran anschließenden Abkühlen der Verbrennungsgase wurde bisher die Cl-Bestimmung durch Titration, durch ionenspezifische Elektroden oder durch Polarografie bzw. amperometrisch vorgenommen. Als Alternative steht auch der Nachweis der Chloride im Strom der die Chloride enthaltenden kondensierten Gase mit einem Integrationsvorgang zur Verfügung. Hierbei handelt es sich jedoch immer noch um ein diskontinuierliches Verfahren, da nur die jeweils zu einer Probe gehörige Chloridmenge - wenn auch über einen längeren Zeitraum sich erstreckend - ermittelt wurde. Hierdurch wurde das bekannte diskontinuierliche Verfahren zusätzlich besonders zeitraubend, da für jede in sich abgeschlossene und begrenzte Probenmenge ein kommpletter Analysenvorgang in Einzelschritten durchlaufen werden muß.

Durch die langwierige Aufbereitung ist der Folgetakt für die einzelnen Analysen (und damit die erzielbare zeitli che Auflösung) beschränkt - selbst wenn eine oder mehrere Personen ausschließlich mit den betreffenden Analysen betraut würden.

Der Erfindung liegt die Aufgabe zugrunde, bei einem Verfahren der eingangs genannten Gattung die Möglichkeit einer kontinuierlichen Analyse in dem Sinne zu schaffen, daß einerseits der Fluß der Substanzen ohne Unterbrechung erfolgt und andererseits das Analysenergebnis mit zeitlich hoher Auflösung Schwankungen der Analyseneingangsgrößen folgen kann.

Diese Aufgabe wird mit den kennzeichnenden Merkmalen des Anspruchs 1 gelöst.

Die Erfindung beruht auf der Erkenntnis, daß die Genauigkeit des kontinuierlichen Analysenergebnisses sich in günstiger Weise dann erhalten läßt, wenn nach einer volumetrisch exakt dosierten kontinuierlichen Probenentnahme, unter Vermischung mit einem Extraktionsmittel der Transport zur weiteren Verarbeitung auf dem Analysenweg ohne zusätzlichen Pumpantrieb in einem geschlossenen System nur durch physikalische Einwirkung von Schwerkraft, Erwärmung (auch nach chemischer Reaktion) bzw. Transport mit einem Trägergas erfolgt. Die analytische Mengenbestimmung wird dann mittels einer Vorrichtung erzeugt, welche durch spezifischen Nachweis eines Anteils in kontinuierlichem Durchlauf zeitnah oder zeitgleich mit hoher Auflösung den Analysenwert ausgibt, wobei wegen der genauen und konstanten Eingangsdosierung eine Rückrechnung auf das Meßvolumen möglich ist. Nur die Ausgangsdosierung der Analysensub stanz muß exakt erfolgen, während die Beimischungen innerhalb eines vorgegebenen Bereichs variabel sind. Dadurch, daß als Reagens Silber in der Elektrolytlösung verwendet wird, welches sich von der Elektrode her durch den verwendeten Regelvorgang selbsttätig ersetzt und somit im wesentlichen unter konstanten Bedingungen zur Verfügung steht, ist keine Unterbrechung für das Ersetzen des Reagens und die Nacheichung der Meßzelle erforderlich. Der Begriff "Extraktion" soll dabei so verstanden werden, daß er sich auf alle Maßnahmen bezieht, bei denen eine Substanz die chlorierten Kohlenwasserstoffe aufnimmt, sei es durch Herauslösung, Anlagerung etc., wie es den eingangs genannten EOX-, AOX- oder auch POX-Verfahren entspricht.

Somit wird die Kontinuität des Stoffflusses an keiner Stelle unterbrochen. Die Auswertung erfolgt in der Tat vollkontinuierlich und nicht - wie bei anderen Verfahren - teil- oder quasikontinuierlich. Die durchschnittliche Verfahrensdurchlaufzeit kann dann gegebenenfalls noch zum genaueren zeitlichen Rückschluß auf den Entnahmezeitpunkt zurückgerechnet werden. Auf diese Weise läßt sich in günstiger Weise ein Betriebsmeßsystem schaffen, welches zuverlässig und, über lange Betriebszeiten auch wartungsfrei, arbeitet.

Vorteilhafte Weiterbildungen der Erfindungen, die auch selbständig nutzbar sind und unabhängig vom hier beschriebenen Kontext und der speziellen Art der Weiterverarbeitung Bedeutung haben, betreffen die Pumpvorrichtung für kontinuierliches feines Dosieren, die Verbrennungskammer für kontinierliche Verbrennung und die coulombmetrische geregelete Meßzelle.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine schematische Darstellung eines Ausführungsbeispiels zur Durchführung des Verfahrens als Betriebsmeßsystem,

Figur 2 ein Detail der Anordnung gemäß Figur 1,

Figur 2a ein Zeitdiagramm zur Erläuterung von Figur 2,

Figur 2b eine Schaltungsausführung sowie

Figur 3 eine Blockdarstellung einer Regelanordnung als Einzelheit der Anordnung gemäß Figur 1.

Eine Rohrleitung 1 wird von dem Meßgut, welches chlorierte Kohlenwasserstoffe (CKW) enthält, durchflossen. Eine Abzweigleitung 2 führt zu einer Filteranordnung 3, welche dazu dient, die zu analysierende Flüssigkeit (Meßgut) von Schwebstoffen zu befreien. Angesaugt und gleichzeitig dosiert wird das Meßgut von einer Pumpe 4, welche weiter unten näher beschrieben werden wird. Die Pumpleistung der Pumpe 4 liegt in der Größenordnung von 2 l/h. Von Bedeutung ist hier, daß die Anordnung so getroffen wird, daß die Pumpwirkung absolut kontinuierlich und stoßfrei in einem weiten Bereich steuerbar von wenigen Millilitern bis einigen Litern pro Stunde zur Verfügung steht. Eine nicht näher dargestellte Pumpe 5 ist an einen Behälter 6 für das Extrahiermittel, in diesem Fall Hexan, angeschlossen. Die Pumpe 5 entspricht in ihrem Aufbau und in ihrer Wirkungsweise der Pumpe 4. Die Ausgangsleitungen 7 und 8 der Pumpen 4 und 5 vereinigen sich vor einer Kapillare 9 als Strömungswiderstand, welche bei einer Länge von 1 bis 10 mm einen Durchmesser von 0,2 bis 2 mm aufweist.

Durch die gleichmäßige Vermischung aufgrund des durch die beiden Pumpen 4 und 5 kontinuierlich aufrechterhaltenen Drucks wird in der Kapillare eine heterogene Suspension des in Wasser befindlichen Meßguts mit Hexan erzeugt. Hexan als Extraktionsmittel bewirkt in einem kontinuierlichen, durchlaufenden Vorgang eine Extraktion der CKW aus der Probenflüssigkeit.

Der Ausgangsanschluß der Kapillare 9 führt in ein Glasgefäß 10, wo der damit gebildete Einlaß kurz oberhalb eines Wasser-Hexan-Spiegels 11 mündet. Das Gefäß 10 ist verbunden mit einer kommunizierenden Röhre 12 mit kleinerem Durchmesser, die mit einem Überlauf für Wasser 13 in Verbindung steht. Hier findet die Abtrennung des Hexans statt. Zur Verringerung der Strömungsgeschwindigkeit ist der Querschnitt des Gefäßes 10 groß gegenüber dem der angeschlossenen Leitungen. Bei einer vorteilhaften - in der Zeichnung nicht dargestellten - Weiterbildung bewirkt ein Magnetrührer innerhalb des Gefäßes 10 durch Erhöhung der Relativgeschwindigkeiten der zu trennenden Partikel (wegen des dann besthenden Geschwindigkeitsgradienten) eine verbesserte Trennung.

Sowohl das Gefäß 10 als auch die Röhre 12 sind nach oben hin mit einer Kapillare 14 bzw. 15 verbunden, die einen Durchlaß zur Umgebungsluft zwecks Entlüftung bildet. Auf diese Weise können sich eventuell bildende Gasblasen entweichen, so daß sich die Flüssigkeitsspiegel in dem Gefäß 10 und dem Rohr 12 in entsprechender Höhe einstellen können.

Durch den Überlauf 13 kann überschüssiges Wasser abfließen.

In dem Gefäß 10 wird das mit dem CKW beladene Hexan (wobei gegebenenfalls noch einige begleitende Substanzen mitgeführt werden können) über eine ebenfalls einen Überlauf bildende Leitung 16 in ein weiteres Gefäß 17 überführt, wo durch Beheizung mit einer Heizvorrichtung 18 eine Überführung in die gasförmige Phase erfolgt. Das mit dem Gefäß 10 verbundene Ende der Leitung 16 bestimmt die Höhe des Hexanspiegels, der aufgrund der unterschiedlichen Gewichte auf gerinfügig anderer Höhe liegt als der Wasserspiegel beim Überlauf 13.

Das andere Ende der Leitung 16 führt innerhalb des Gefäßes 17 auf eine Glas- oder Keramikfritte 19, und endet als Kapillare 20 ausgezogen. Die Fritte 19 ist bevorzugt zweiteilig ausgebildet, wobei in einem Zwischenraum inerte Körperchen als Füllmaterial zur Oberflächenerhöhung und thermischen Pufferung vorgesehen sind.

Aus einer Druckflasche 21 wird über ein Reduzierventil 22 Stickstoff zugeführt und in einer Menge einstellbar in einem Bereich von ca. 5 bis 100 l/h an der Heizung 18 vorbei von unten her durch die Fritte geleitet. Die Heizleistung der Heizung 18 ist einstellbar, wobei der Ausgang einer Stromquelle 23 über ein Stellglied 24 einstellbar ist, so daß dem als ohmscher Widerstand ausgebildeten Heizelement 18 unterschiedliche elektrische Leistungen zugeführt werden können. Damit läßt sich eine Erhitzung bis 500° C erzielen.

Durch die Einstellung der Temperatur lassen sich leichtflüchtige oder schwerer verdampfbare Anteile selektiv auswählen, so daß die für die nachfolgende kontinuierliche Verbrennung auszuwählenden Anteile selektiv vorwählbar sind.

Wird auf eine Beheizung vollständig verzichtet, so liegt die Ausgangstemperatur unterhalb der Raumtemperatur, da dadurch die Verdampfungswärme entzogen wird. Durch eine Leitung 25, welche an das Gefäß 17 oben anschließt, wird das Gasgemisch einer Anordnung 26 zugeleitet, in der eine kontinuierliche Verbrennung stattfindet.

In die Anordnung 26 wird über einen Stutzen 27 $O_2$ eingeleitet, so daß es sich mit dem Gasvolumen der ebenfalls von unten her die Anordnung 26 erreichenden Leitung 25 vermischt. Zwischen zwei Fritten 28 und 29 ist ein feines inertes Pulver mit hoher Wärmekapazität vorgesehen. Die Strömungsgeschwindigkeit des $O_2$ ist groß gewählt, so daß sich kein explosives Gemisch bilden kann. Die Explosionsgefahr wird zusätzlich durch die Wärmekapazität des inerten Pulvers herabgesetzt. Die Mischung der beiden zugeführten Gaskomponenten erfolgt in einer Düse 30, welche in einen Flammenraum mündet. Seitlich in diesen Flammenraum hinein ragen zwei Edelstahl- oder Wolframelektroden 31 und 32, welche mit einer Hochspannungszündanlage 33 verbunden sind. Die Hochspannungs(transistor)zündanlage entspricht einer Kraftfahrzeugzündanlage, die mit 50 Hz Netzfrequenz betrieben wird. Diese Zündanlage wird immer dann aktiviert, wenn eine Fotozelle 34 Dunkelheit im Flammenraum feststellt. Durch die von der ordnungsgemäß brennenden Flamme ausgehende Lichtstrahlung wird also der Innenwiderstand der Fotozelle 34 herabgesetzt, wodurch die Zündanlage mittels entsprechender elektronischer Bauelemente stillgesetzt wird. Dieses Prinzip entspricht beispielsweise demjenigen wie es bei Ölbrennern im Haushaltsbereich eingesetzt wird. Die Zündung erfolgt mit einer gewissen Zeitüberlappung, d.h. der Zündvorgang wird gesteuert durch ein Zeitglied, wenn die Flamme bereits entzündet ist, um eine stabile Verbrennung zu erzielen. Die Flamme brennt bereits unterhalb der Fritten, so daß hier eine hohe Vermischung erzielt wird.

Durch die Verbrennung erfolgt eine Umwandlung in anorganische Chloride (Mineralisierung).

Die aus der Anordnung 26 über einen Auslaß 35 entweichenden Gase werden einem mit Wasser durchflossenen Kühler 36 zur Abkühlung und Kondensation zugeführt. Dieser Kühler kann gegebenenfalls mehrteilig ausgebildet sein, wobei die Auslegung derart erfolgen muß, daß insbesondere die Heizwirkung des Heizelements 18 auch bei höchster Heizstufe kompensiert und eine Erwärmung der nachfolgenden Meßzelle vermieden wird.

Über eine Leitung 39 gelangt das Verbrennungsprodukt zu einer Cl-Detektionseinrichtung 40, welche als Cl-spezifischer Detektor eine vollkontinuierliche Cl-Detektion ermöglicht, ohne daß phosphororganische oder schwefelorganische Verbindungen bzw. Pestizide stören. Wäre statt dessen ein HCl-Nachweis vorgesehen, so würde beispielsweise die hier entsprechend anfallende Salpetersäure das Meßergebnis verfälschen.

Als Cl-spezifischer Detektor wird ein Mikrocoulomb-Meter als Analyseteil verwendet. In einem geeigneten ELektrolyten sind zwei Paare von Silberelektroden 41 und 42 bzw. 43 und 44 vorgesehen. Weiterhin vorhanden ist eine Rührvorrichtung 45, welche eine gleichmäßige Durchmischung des Elektrolyten bewirkt.

Der Elektrolyt besteht aus konzentrierter Essigsäure mit einer geringfügigen Beimengung von Silbernitrat, um die Leitfähigkeit des Elektrolyten für die nachfolgend dargestellte Regelung zu gewährleisten.

Während die erste Elektrodengruppe mit den Elektroden 41 und 42 an dem Stromausgang 46 einer Steuereinheit 47 angeschlossen ist, bilden die Elektroden 43 und 44 Potentialmeßelektroden die eine Spannung liefern, welche an einen Meßeingang 48 der Steuereinheit 47 gelangt.

Die Anordnung funktioniert nun folgendermaßen: Die in den Elektrolyten eingeleiteten Cl-Ionen bewirken, daß AgCl gebildet wird, wobei die gebundenen Silberionen von dem Elektrodenpaar 41 und 42 (je nach Polarität) nachgeliefert werden. Der sich einstellende Strom ist dabei ein Maß für die pro Zeiteinheit eintreffenden Chlorid-Ionen. Der Vorgang verläuft stöchiometrisch 1:1 und der Strom ergibt sich aus dem Faradayschen Gesetz. Je nach Cl-Konzentration lassen sich Stromstärken vor $10^{-7}$ bis $10^{-1}$ Ampère auswerten. Bei der Anordnung ist von Bedeutung, daß der Strom so lange nachgeliefert wird, bis das Potential an den Meßelektroden 43 und 44 ausgeglichen ist. Da die Umverteilung des Elektrolyten Zeitkonstanten und gegebenenfalls auch Totzeiten mit sich bringt, wurde eine Regelanordnung geschaffen, welche es ermöglicht, den Meßwert in möglichst kurzer Zeit auch bei stark schwankenden Konzentrationsänderungen kontinuierlich zu ermitteln, wobei durch die dargestellte Regelung der sich aktuell einstellende Strom weitgehend der jeweils vorliegenden Ionenkonzentration entspricht.

Über einen konstanten Zulauf 49 und einen kontrollierten Abfluß 50 wird die Elektrolytmenge bei ca. 20 ml konstant gehalten, der Elektrolyt aber kontinuierlich ausgetauscht und Nebenprodukte abgeführt.

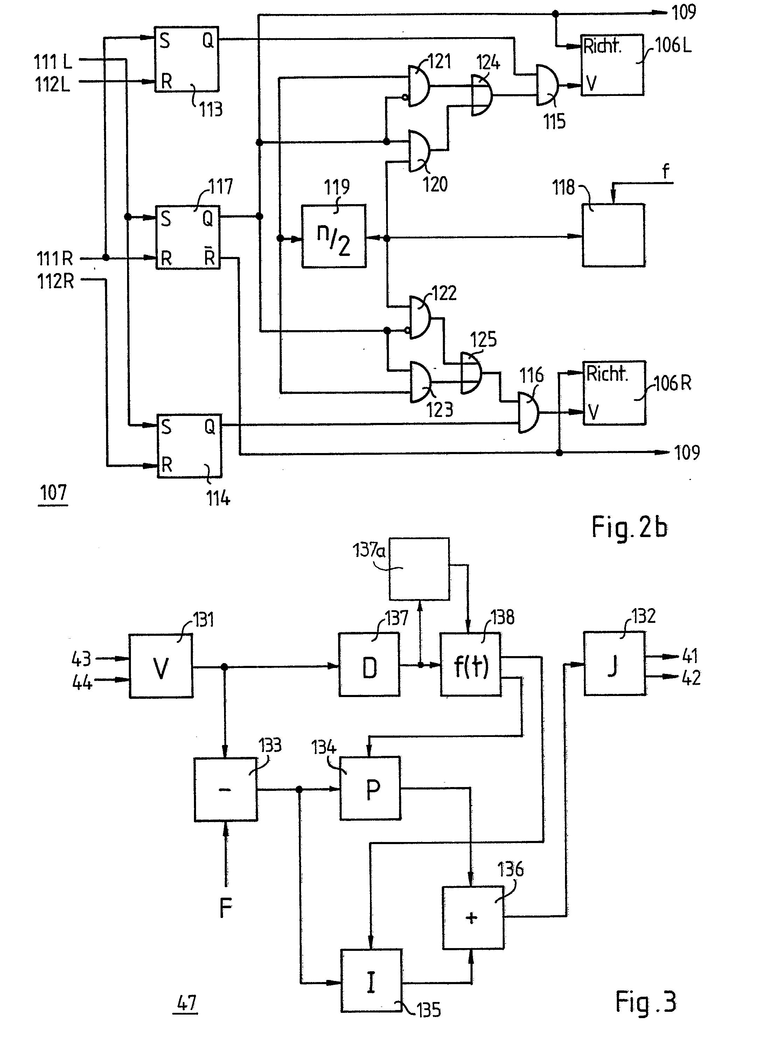

Das Steuergerät 47 ist im einzelnen in Figur 3 dargestellt und wird anhand dieser Figur näher beschrieben.

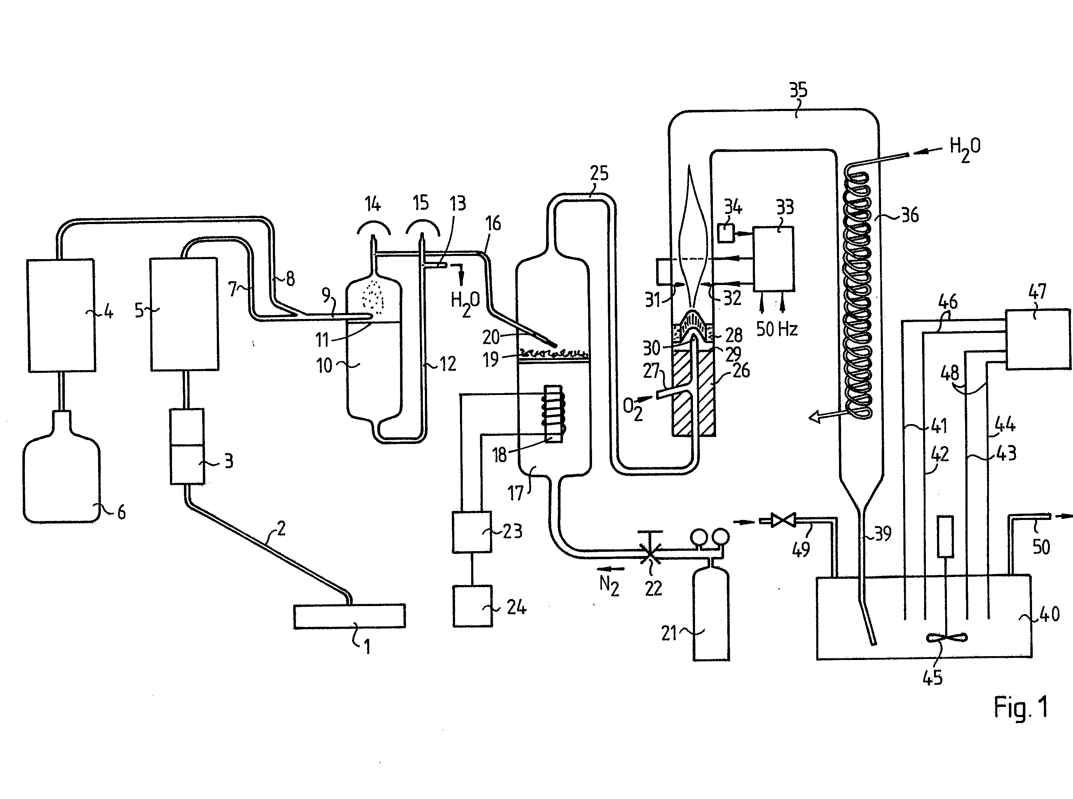

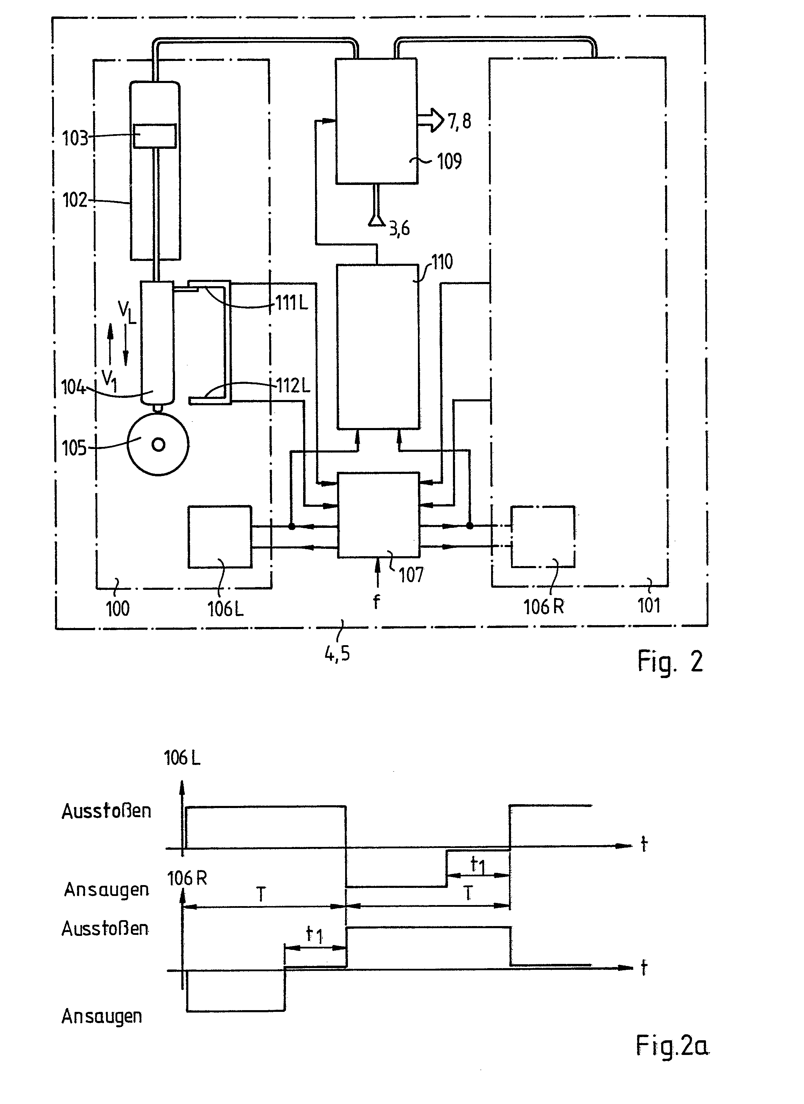

In den Figuren 2 bis 2b ist die Pumpenanordnung zur hochpräzisen Förderung von Analysensubstanz oder von Lösungsmittel dargestellt, wie sie in Figur 1 mit 4 bzw. mit 5 bezeichnet ist. Diese Anordnung besteht aus zwei Kolbenpumpenanordnungen 100 und 101, welche identisch aufgebaut sind, wobei lediglich die Pumpe 101 schematisch in Einzel heiten wiedergegeben ist. Sie weist einen Zylinder 102 auf, in dem ein Kolben 103 beweglich gelagert ist. Der Kolben ist in beiden Richtungen antreibbar mittels eines Spindelantriebs 104, der über ein Schneckengetriebe 105 von einem Schrittmotor 106 (L bzw. R für "links" und "rechts") bewegt wird. Dieser Schrittmotor 106 wird mit Steuerimpulsen versorgt von einer Steuereinheit 107. Diese Steuereinheit bestimmt durch die Impulsfrequenz und -folge, das Fördervolumen der Pumpeinheit insgesamt und die Taktzeiten und Richtungen der einzelnden Kolbenpumpen.

Das Zeitdiagramm der Arbeitsweise der beiden Pumpenteile 100 und 101 ist in Figur 2a wiedergegeben, wobei der Arbeitshub "Ausstoßen" in positiver Richtung nach oben und das Ansaugen nach unten dargestellt ist. Diese Wiedergabe entspricht sinngemäß der Bewegungsrichtung der Kolben in Figur 2. Eine Magnetventilverteileranordnung 109 wird von einer Steueranordnung für die Ventile 110 angesteuert, wobei die Magnetventile derart beeinflußt werden, daß während des Ansaugvorgangs der Weg von der Leitung 6 zum Zylinder hin geöffnet ist, während in der Ausstoßphase der Zylinder mit den Leitungen 7 bzw. 8 verbunden ist.

Von besonderer Bedeutung ist in diesem Zusammenhang, daß die Taktfolge zur Ansteuerung des Schrittmotors 106L (bzw. des entsprechenden Schrittmotors 106R in der Einheit 101) beim Ansaugen mit einer relativ erhöhten Impulsfolge vorgenommen wird, so daß dieser Vorgang weniger Zeit braucht als der Ausstoßvorgang. Die beiden Pumpen 100 und 101 arbeiten im Gegentakt, wobei der Ansaugvorgang der jeweils anderen Pumpe um eine Zeitdauer $t_1$ vor dem Ende des jewei ligen Taktintervalls T beendet ist, so daß die Übernahme des Ausstoßtaktes ohne Zeitverzögerung und gegebenenfalls mit einem kleinen Zeitintervall überlappend vorgenommen werden kann, und keine Stöße im Druckaufbau in der Auslaßleitung 7 bzw. 8 entstehen und eine kontinuierliche Pumpleistung bei genauer Dosierung zur Verfügung steht.

Das Verhältnis $t_1$ zu T kann als konstanter Quotient vorgesehen sein oder aber es kann die Zeitdauer $t_1$ auch eine feste Zeiteinheit bilden, welche jedoch in jedem Fall kleiner als die Zeitdauer T sein muß. Bei der hier dargestellten Ausführung wird durch entsprechende Heraufsetzung der Taktimpulse während des Ansaugvorgangs dieser Takt gegenüber dem Ausstoßtakt verkürzt, so daß die Förderleistung der Pumpen durch die Beeinflussung der Taktimpulse der Einheit 107 an einem einzigen Stellglied verändert werden kann. Die Leistung der Pumpen ist dabei proportional der Impulsfrequenz. Die Umschaltung von einer Pumpe auf die andere bei Beendigung des Ausstoßvorgangs wird dabei durch ein oberes Fühlelement 111 bewirkt, während das untere Fühlelement 112 jeweils die Impulsabgabe an den Antriebsmotor 106 unterbricht, wenn der Kolben seine untere Wegbegrenzung erreicht hat. Die obere Wegbegrenzung schaltet also die Antriebsrichtungen beider Pumpen um, während das untere Fühlelement 112 jeweils nur die zugeordnete Pumpe stillsetzt. Mit der Umkehrung der Antriebsrichtung jeder Pumpe ist die dargestellte Geschwindigkeitsheraufsetzung verbunden.

In Figur 2b ist eine Steuerschaltung für die Antriebsmotore 106L sowie 106R einschließlich Magnetventilsteuerung 109 dargestellt. Zwei Latches 113 bzw. 114 sind jeweils den Motoren 106L bzw. 106R zugeordnet. Ihr Ausgangssignal Q schaltet den zugeordneten Motor ein, wenn das Latch gesetzt ist, d.h. ein logischen "H"-Signal am Ausgang Q anliegt. Das Ausgangssignal dieser Ausgänge Q ist mit jeweils zugeordneten UND-Gattern 115 bzw. 116 verbunden, an deren weiterem Eingang ein die Drehzahl des zugeordneten Motors bestimmendes Taktsignal anliegt, dessen Erzeugung weiter unten näher dargestellt werden soll. Das Ausgangssignal der Latches 113 bzw. 114 bestimmt jetzt der UND-Gatter 115 bzw. 116, ob dieses Taktsignal zur Ansteuerung des jeweiligen Schrittmotors durchgelassen wird. Der obere Endabschalter 111L bzw. 111R schaltet bei seinem Erreichen jeweils die Drehrichtung um (S- bzw. R-Eingänge des Geschwindigkeitsumschalt-Latches 117). Weiterhin startet der obere Endabschalter 111L bzw. 111R jeweils bei seinem Erreichen den dem anderen Kolben zugeordneten Schrittmotor 106L bzw. 106R. Die Drehrichtung wird durch ein Drehrichtungssignal bestimmt, welches der Ausstoßrichtung der jeweiligen Kolbenpumpe zugeordnet ist. Die entsprechende Steuersignale gelangen von den Ausgängen Q bzw. $\bar{\bar{Q}}$ zu den entsprechenden Steuereingängen der Schrittmotoren 106L bzw. 106R. Der jeweilige Motor wird durch einen unteren Endabschalter 112L bzw. 112R stillgesetzt, wenn dieser erreicht wird und die Pumpe kurz vor der Drehrichtungsumkehr nach Beendigung des Ansaugtaktes wieder zum Ausstoßen bereit ist.

Diese Betriebsweise stimmt mit den in den Figuren 2a dargestellten Zeitdiagrammen überein.

Zusätzlich zu beschreiben ist jetzt noch die Takterzeugung für die Schrittmotoren 106L bzw. 106R. Das Taktsignal geht aus einem Taktgenerator 118, dessen Frequenz S über ein Steuereingang stufenlos einstellbar ist und somit die Förderleistung der Pumpen über einen weiten Bereich präzise regelbar macht. Das Ausgangssignal des Taktgebers 118 gelangt zu einem Frequenzteiler 119, wo es um einen Faktor von beispielsweise zwei herabgesetzt ist.

Dieses niedrigere Taktsignal bestimmt die Arbeitsgeschwindigkeit, während der Ansaughub mit der erhöhten Taktfrequenz gefahren wird. Die ungeteilte bzw. die geteilte Taktfrequenz gelangen zu UND-Gattern 120 bzw. 121 für den Motor 106L bzw. 122 und 123 für den Motor 106R. Die Ausgänge der Gatter 120, 121 sind mit den Eingängen eines ODER-Gatters 124 und die Ausgänge der UND-Gatter 122, 123 mit einem ODER-Gatter 125 verbunden, welche diese Ausgangssignale verknüpfen und jeweils einem weiteren Eingang der UND-Gatter 115 bzw. 116 zuleiten.

Je nach Stellung des Richtungs-Latches 117 wird eines der UND-Gatter 120 bzw. 121 sowie 122 bzw. 123 durchlässig geschaltet. Bei aktiviertem Q-Signal des Latches 117 ist das das UND-Gatter 120 bzw. 123 und in dieser Stellung läuft der Motor 106R mit reduzierter Ausstoßgeschwindigkeit, während der Motor 106L mit der ungeteilten Taktfrequenz ansaugt.

In der anderen Stellung des Latches 117 (Q = "L") werden die UND-Gatter 121 bzw. 122 über ihre invertierenden Eingänge durchlässig geschaltet und die Geschwindigkeits verhältnisse kehren sich wegen der dann vertauschten Taktansteuerung um. Auf diese Weise kehren die Antriebsmotoren nach beendetem Ausstoßhub bei Erreichen des oberen Endschalters des ausstoßenden Kolbens 111L bzw. 111R ihre Antriebsrichtung um, der wartende Kolben geht ohne zeitliche Unterbrechung zum Ausstoßen über, während der erstgenannte Kolben mit erhöhter Antriebsgeschwindigkeit einen neuen Ansaugvorgang einleitet bis er den unteren Endschalter 112L bzw. 112R erreicht, wo er stillgesetzt wird und darauf wartet, bis der andere Kolben den Austauschvorgang beendet. Die Magnetventilsteuerung 109 wird ebenfalls über die Ausgangsleitungen des Latches 117 in Figur 2b angesteuert, wobei die Umschaltung der Magnetventile von Ansaug-Richtung in Ausstoß-Richtung entsprechend der Drehrichtungssteuerung der Motoren vorgenommen wird, so daß die Ventile synchron ansprechen. Durch die Verwendung von elektronisch angesteuerten Magnetventilen werden die Ventilbetätigungszeiten derart reduziert, daß sie im Prozeß nicht störend in Erscheinung treten, d.h. keine den Prozeß beeinflussenden Impulse verursachen.

In Figur 3 ist die Meßzellenregelung näher dargestellt. In der Meßzelle, die vom Meßgut kontinuierlich durchströmt wird, sind die vier Elektroden 41 bis 44 vorgesehen. Die Potential-Meßelektroden 43, 44 sind mit dem Eingang eines Meßverstärkers 131 verbunden, während die zwei weiteren Elektroden 41 und 42 als Generatorelektroden, an dem Ausgang einer steuerbaren Stromquelle 132 angeschlossen sind. Das Ausgangssignal des Verstärkers 131 wird bestimmt durch die Differenz der Ausgangsspannungen der Elektroden 43, 44. Dieses Signal wird zusammen mit einer von außen her beeinflußbaren Führungsgröße F einer Subtrahierschaltung 133 zugeführt, welche die Differenz einer Führungsgröße und des Ausgangssignales des Meßverstärkers bildet. Diese Differenz als Ausgangssignal des Subtrahierers 133 gelangt zu den Eingängen eines frequenzunhabhängigen Übertragungsglieds 134 mit proportionaler oder nichtlinearer Kennlinie und ferner parallel zum Eingang eines Integrators 135. Die Ausgangssignale des frequenzunabhängigen und des integrierenden Glieds 134 bzw. 135 werden in einem Additionsglied 136 addiert und als Stellsignal dem Eingang der steuerbaren Stromquelle 132 zugeführt.

Die Parameter des frequenzunhabhängigen und des integrierenden Gliedes 134 bzw. 135 werden durch das Ausgangssignal eines differenzierenden Glieds 137 gesteuert, welches ebenfalls an den Augang des Meßverstärkers angeschlossen ist. Diese Parameter werden nach einem mittels eines Funktionsgliedes 138 einstellbaren funktionalen Zusammenhang derart gesteuert, daß der von der Stromquelle erzeugte Strom angenähert ein Maß für die Stoffkonzentration bildet. Die Umrechnung erfolgt arithmetisch oder durch Adressieren einer entsprechenden als ROM-Speicher ausgebildeten "Tabelle".

Die Regelparameter des so gebildeten Reglers werden derart eingestellt, daß die durch die Eingangselektroden festgestellten Konzentrationsänderungen möglichst kurzfristig ohne "Überschwingen" ausgeglichen werden, da eine Überdosierung von Silber im Elektrolyten nicht wie bei anderen technischen Reglern durch eine entgegengesetzte Ansteuerung der Generatorelektroden ausgeglichen werden kann. Hier kann der Ausgleich nur durch den kontinuierlichen Abtransport des Elektrolyten erfolgen. Es hat sich jedoch gezeigt, daß es günstig ist, mit größer werdendem D-Anteil den I-Anteil zu verkleinern, so daß bei starken Konzentrationsschwankungen beim Anfahren des Systems zunächst ein Ausgleich im wesentlichen uber den P-Anteil erfolgt. Bei eingefahrenem System und geringeren Konzentrationsschwankungen können diese bei erhöhtem I-Anteil jedoch zügig ausgeregelt werden, wobei eine bleibende Regelabweichung dann ohne "überschwingen" kompensiert wird.

In Figur 3 ist die Meßzellenregelung näher dargestellt. In der Meßzelle, die vom Meßgut kontiunierlich durchströmt wird, sind vier Elektroden 41 bis 44 vorgesehen. Die Potential-Meßelektroden 43, 44 sind mit dem Eingang eines Meßverstärkers 131 verbunden, während die zwei weiteren Elektroden 41 und 42 als Generatorelektroden, an dem Ausgang einer steuerbaren Stromquelle 132 angeschlossen sind. Das Ausgangssignal des Verstärkers 131 wird bestimmt durch die Differenz der Ausgangsspannungen der Elektroden 43, 44. Dieses Signal wird zusammen mit einer von außen her beeinflußbaren Führungsgröße F einer Subtrahierschaltung 133 zugeführt, welche die Differenz einer Führungsgröße und des Ausgangssignales des Meßverstärkers bildet. Diese Differenz als Ausgangssignal des Subtrahierers 133 gelangt zu den Eingängen eines frequenzunhabhängigen Übertragungsglieds 134 mit proportionaler oder nichtlinearer Kennlinie und ferner parallel zum Eingang eines Integrators 135. Die Ausgangssignale des frequenzunabhängigen und des integrierenden Glieds 134 bzw. 135 werden in einem Additionsglied 136 addiert und als Stellsignal dem Eingang der steuerbaren Stromquelle 132 zugeführt.

Die Parameter des frequenzunhabhängigen und des integrierenden Gliedes 134 bzw. 135 werden durch das Ausgangssignal eines differenzierenden Glieds 137 gesteuert, welches ebenfalls an den Augang des Meßverstärkers angeschlossen ist. Diese Parameter werden nach einem mittels eines Funktionsgliedes 138 einstellbaren funktionalen Zusammenhang derart gesteuert, daß der von der Stromquelle erzeugte Strom angenähert ein Maß für die Stoffkonzentration bildet. Die Umrechnung erfolgt arithmetisch oder durch Adressieren einer entsprechenden als ROM-Speicher ausgebildeten "Tabelle". Bei einer vorteilhaften Ausführung wird der funktionale Zusammenhang in Abhängigkeit von der Polarität des Ausgangssignals des D-Glieds 137 umgeschaltet. Hierfür ist ein Polaritätsdiskriminator 137a vorgesehen, dessen Ausgangssignal ein Auswahlsignal für im Speicher 138 enthaltene Regelfunktionen bildet. Damit lassen sich die "Parametersätze" des Reglers umschalten. Hierdurch wird der Tatsache in optimaler Weise Rechnung getragen, daß die Regelung der Meßzelle lediglich durch den Zufluß des Elektrolyten beeinflußt werden kann. Bei Überschuß von Elektrolyt sorgt ein schnelles Eingreifen des Reglers für eine umgehende Drosselung. Da eine Abfuhr des Elektrolyten nur in dem Maße des "natürlichen" Abtransports im Zuge der Analyse erfolgen kann, ist hier eine verfeinerte Regelung nicht möglich. Bei Elektrolytenmangel hingegen ist muß die Zugabe derart dosiert erfolgen, daß ein "Überschwingen" vermieden ist. Hier sorgt der I-Anteil für einen ausgeglichenen Zustand ohne bleibende Regelabweichung.

Die Regelparameter des so gebildeten Reglers werden derart eingestellt, daß die durch die Eingangselektroden festge stellten Konzentrationsänderungen möglichst kurzfristig ohne "Überschwingen" ausgeglichen werden, da eine Überdosierung von Silber im Elektrolyten nicht wie bei anderen technischen Reglern durch eine entgegengesetzte Ansteuerung der Generatorelektroden ausgeglichen werden kann. Hier kann der Ausgleich nur durch den kontinuierlichen Abtransport des Elektrolyten erfolgen. Es hat sich jedoch gezeigt, daß es günstig ist, mit größer werdendem D-Anteil den I-Anteil zu verkleinern, so daß bei starken Konzentrationsschwankungen beim Anfahren des Systems zunächst ein Ausgleich im wesentlichen über den P-Anteil erfolgt. Bei eingefahrenem System und geringeren Konzentrationsschwankungen können diese bei erhöhtem I-Anteil jedoch zügig ausgeregelt werden, wobei eine bleibende Regelabweichung dann ohne "überschwingen" kompensiert wird.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Insbesondere beschränkt sich die Ausführung nicht auf die Realisierung mit diskreten logischen Baugruppen, sondern läßt sich vorteilhaft auch mit programmierter Logik - vorzugsweise unter Verwendung eines Mikroprozessors - realisieren.

**Patentansprüche**

1. Verfahren zur analytischen Bestimmung des Gehalts an chlorierten, gegebenenfalls entsprechend auch bromierten oder jodierten, Kohlenwasserstoffen (CKW), in einer zu analysierenden Flüssigkeit,
**gekennzeichnet durch**
die Zuführung der Flüssigkeit in ununterbrochenem Fluß unter Vermischung mit einem, insbesondere organischen, Extraktionsmittel unter dosierender, kontinuierlicher und stoßfreier Pumpwirkung,
die nachfolgende Phasentrennung, Vergasung, Verbrennung sowie Kondensation des organischen Anteils,
die quantitative kontinuierliche Bestimmung des mit den nachzuweisenden CKWs in stöchiometrischem Verhältnis stehenden Chlors durch Einleitung in eine coulombmetrische Meßzelle, sowie
die Rückrechnung vom jeweils für einen Bezugszeitraum ermittelten Chlor auf die in dem entsprechenden Bezugszeitraum angefallenen chlorierten Kohlenwasserstoffe.

2. Verfahren, insbesondere nach Anspruch 1, **da durch gekennzeichnet,** daß die Vermischung der zu analysierenden Flüssigkeit mit dem organischen Extraktionsmittel durch zwei separat die Flüssigkeit und das Extraktionsmittel kontinuierlich und stoßfrei fördernde Pumpen unter gemeinsamer Einleitung in eine nachgeschaltete Kapillare als Strömungswiderstand erfolgt.

3. Verfahren nach einem der vorangehenden Ansprüche, **dadurch gekennzeichnet,** daß jede Pumpe durch zwei Kolbenpumpen gebildet wird, deren Auslässe über magnetisch betätigbare Ventile, die abwechselnd jeweils koordiniert mit dem Austoßhub der Kolbenpumpen öffnen, zu einer gemeinsamen Leitung führen, wobei am Ende des Ausstoßhubs ein Schalter vorgesehen ist, welcher jeweils die andere Pumpe in Ausstoßrichtung einschaltet.

4. Verfahren nach Anspruch 3, **dadurch gekennzeichnet,** daß der Ansaughub zeitlich kürzer bemessen ist als der Austoßhub, so daß nach dem Ansaughub, insbesondere durch einen am Ende des Ansaughubs vorgesehenen Schalter, die betreffende Pumpe stillgesetzt wird bis die andere ihren Ausstoßhub beendet hat, wobei insbesondere mit der Drehrichtungsumschaltung der Kolbenpumpen eine Geschwindigkeitsumschaltung erfolgt in der Weise, daß die Geschwindigkeit in Ansaugrichtung größer ist als die Geschwindigkeit in Austoßrichtung.

5. Verfahren nach einem der Ansprüche 3 oder 4, **dadurch gekennzeichnet,** daß die Kolbenpumpen durch elektrische Schrittmotoren angetrieben sind, wobei die Geschwindigkeit der Pumpen durch die veränderbare Impulsfrequenz der Schrittmotoren bestimmt wird, und/oder der Antrieb der Kolbenpumpen über eine Spindelführung erfolgt.

6. Verfahren nach einem der vorangehenden Ansprüche, **dadurch gekennzeichnet,** daß als Extraktionsmittel Hexan verwendet wird.

7. Verfahren nach einem der vorangehenden Ansprüche, **dadurch gekennzeichnet,** daß zur Phasentrennung zwei kommunizierende Gefäße mit unterschiedlichem Querschnitt verwendet werden, wobei die Einleitung in das Gefäß mit größerem Querschnitt oberhalb des Spiegels der Trennschicht erfolgt und die oberen Bereiche der Gefäße Überläufe aufweisen, von denen derjenige für das Gefäß mit größerem Durchmesser mit einer Leitung zur Weiterleitung des organischen Extraktionsmittels in Verbindung steht, während derjenige des Gefäßes mit kleinerem Durchmesser mit dem Ablauf der restlichen Flüssigkeit in Verbindung steht.

8. Verfahren nach Anspruch 7, **dadurch gekennzeichnet,** daß im oberen Bereich des Gefäßes eine Entlüftung vorgesehen ist, welche insbesondere aus einer Kapillare besteht.

9. Verfahren nach einem der vorangehenden Ansprüche, **dadurh gekennzeichnet,** daß die Vergasung durch Beheizung mittels eines Heizkörpers, insbesondere mittels eines ohmschen Widerstandes, erfolgt.

10. Verfahren nach Anspruch 9, **daduch gekennzeichnet,** daß das abgeleitete Extraktionsmittel auf eine Fritte geleitet wird, die in Richtung Ableitung von einem inerten Gas, insbesondere N2, durchströmt wird, welches eine Beimengung bildet.

11. Verfahren nach Anspruch 10, **dadurch gekennzeichnet,** daß das inerte Gas vor dem Passieren der Fritte den Heizkörper umströmt.

12. Verfahren nach einem der Ansprüche 10 oder 11, **dadurch gekennzeichnet,** daß die Fritte einen Körper bestehend aus einem Material mit großer Wärmekapazität aufweist.

13. Verfahren, insbesondere nach einem der vorangehenden Ansprüche, **dadurch gekennzeichnet,** daß die kontinuierliche Verbrennung nach $O_2$-Beimischung erfolgt.

14. Verfahren nach Anspruch 13, **dadurch gekennzeichnet,** daß die Verbrennung im Bereich einer Fritte erfolgt, die fein verteilte Körper aus inertem Material enthält.

15. Verfahren nach einem der Ansprüche 13 oder 14, **dadurch gekennzeichnet,** daß eine Hochspannungs-, insbesondere Transistor-, Zündeinrichtung vorgesehen ist, welche bei verlöschender Flamme mittels Dunkelheitsdetektor selbsttätig aktiviert wird, welche insbesondere in den Brennraum oberhalb einer Düse hineinragende Edelstahl- oder Wolfram-Elektroden aufweist.

16. Verfahren nach einem der vorangehenden Ansprüche, **dadurch gekennzeichnet,** daß nach der Verbrennungsstufe eine Kühlvorrichtung zur Kondensation der Verbrennungsgase vorgesehen ist, welche Wasserkühlung mittels Kühlschlangen aufweist.

17. Verfahren nach einem der vorangehenden Ansprüche, **dadurch gekennzeichnet,** daß die coulombmetrische Meßzelle Silberelektroden aufweist, wobei der bei Nachlieferung von Silberionen in den Elektrolyten fließende elektrische Strom ein Maß für die Menge des mit dem Silber im Elektrolyten reagierenden Chlors ist.

18. Verfahren nach Anspruch 17, **dadurch gekennzeichnet,** daß der Elektrolyt aus konzentrierter Essigsäure mit Silbernitratbeimischung zur Erzeugung eines definierten Potentials an Potentialmeßelektroden auch bei fehlendem Chlor besteht.

19. Verfahren insbesondere nach Anspruch 17, **dadurch gekennzeichnet,** daß eine Anordnung zur kontinuierlichen Erneuerung des Elektrolyten unter Aufrechterhaltung einer vorbestimmten Elektrolytmenge, insbesondere unter Verwendung eines Rührers, vorgesehen ist.

20. Verfahren nach einem der vorangehenden Ansprüche, **dadurch gekennzeichnet,**
daß in der Meßzelle, die vom Meßgut kontiunierlich durchströmt wird, vier Elektroden vorgesehen sind,
daß zwei der vier Elektroden Potential-Meßelektroden bilden und mit dem Eingang eines Meßverstärkers verbunden sind und
daß die zwei weiteren Elektroden Generatorelektroden bilden, deren Signal dem Ausgang einer steuerbaren Stromquelle zugeführt wird, die vom Ausgangssignal des Meßverstärkers gesteuert wird, wobei der von der Stromquelle erzeugte Strom angenähert ein Maß für die Stoffkonzentration bildet.

21. Verfahren nach Anspruch 20, **dadurch gekennzeichnet,** daß die Differenz einer Führungsgröße und des Ausgangssignales des Meßverstärkers gebildet wird, die insbesondere den Eingängen eines frequenzunhabhängigen Übertragungsglieds mit proportionaler oder nichtlinearer Kennlinie und ferner parallel dazu einem Integrator zugeführt wird.

22. Verfahren nach Anspruch 21, **dadurch gekennzeichnet,** daß mindestens ein Parameter des frequenzunhabhängigen und/oder des integrierenden Gliedes gesteuert werden in Abhängigkeit vom Ausgangssignal, und insbesondere von der Polarität des Ausgangssignals, eines differenzierenden Glieds, welches an den Ausgang des Meßverstärkers angeschlossen ist, wobei insbesondere die Ausgangssignale des frequenzunabhängigen und des integrierenden Glieds addiert und dann als Stellsignal dem Eingang der Stromquelle zugeführt werden.

23. Verfahren nach einem der vorangehenden Ansprüche, **dadurch gekennzeichnet,** daß im Verfahrensweg Fühlelemente als Teil einer Sicherheitsschaltung vorgesehen sind, wobei bei Abweichung von einem jeweils vorgegebenen Sollwert bezüglich Stoffluß, Temperatur, Flammenintensität etc. mittels einer ODER-Verknüpfung das Verfahren unterbrochen wird.

EP 0 316 260 A2

$H_2O$

$H_2O$

50 Hz

$O_2$

$N_2$

Fig. 1

HAR 37.1

103

102

$V_L$

$V_1$

104

105

111L

112L

100    106 L    f    107    106 R    101

4,5

110

7, 8

109

3,6

Fig. 2

106 L

Ausstoßen

Ansaugen

106 R

Ausstoßen

Ansaugen

T    $t_1$    T

$t_1$

t

t

Fig. 2a

HAR 37.1

Fig. 2b

Fig. 3